# EUROPÄISCHE PATENTSCHRIFT

(11) **EP 2 350 033 B1**
(45) Veröffentlichungstag und Bekanntmachung des Hinweises auf die Patenterteilung: **19.09.2012**
(21) Anmeldenummer: 09783673.8
(22) Anmeldetag: 02.10.2009
(51) Int. Cl.: C07D 301/10

(54) **VERFAHREN ZUR HERSTELLUNG EINES ALKYLENOXIDS**
METHOD FOR PRODUCING AN ALKYLENE OXIDE
PROCÉDÉ DE FABRICATION D UN OXYDE D ALKYLÈNE

(30) Priorität: 08.10.2008 EP 08166057
(43) Veröffentlichungstag der Anmeldung: 03.08.2011
(73) Patentinhaber: BASF SE, 67056 Ludwigshafen (DE)
(72) Erfinder: GITTER, Markus, 1160 Brüssel (BE)
(86) Internationale Anmeldenummer: PCT/EP2009/062804
(87) Internationale Veröffentlichungsnummer: WO 2010/040688

(56) Entgegenhaltungen:
- EP-A2- 0 101 008
- DE-A1- 2 209 392
- DE-B1- 2 519 599

## Beschreibung

Die vorliegende Erfindung betrifft ein kontinuierliches Verfahren zur Herstellung eines Alkylenoxids durch Direktoxidation eines Alkens mit Sauerstoff an einem Silber enthaltenden Katalysator, wobei das Verfahren eine integrierte Behandlung des Katalysators zur Erhöhung seiner Aktivität und/oder Selektivität beinhaltet. Ebenso betrifft die Erfindung die Verwendung eines solchen Verfahrens zur Regenerierung eines gebrauchten, bereits zur Alkylenoxid-Herstellung verwendeten Katalysators.

Alkylenoxide sind wichtige Grundchemikalien, die vielseitige Anwendungen finden. Zur Herstellung von Alkylenoxiden sind im Stand der Technik verschiedene Herstellungsverfahren grundsätzlich bekannt. Alkylenoxide können beispielsweise durch Umsetzung von Olefinen mit Hydroperoxiden, z.B. mit Wasserstoffperoxid, oder durch Direktoxidation mit Sauerstoff hergestellt werden.

Ethylenoxid wird industriell häufig durch Direktoxidation von Ethylen mit Sauerstoff in Gegenwart von Silber enthaltenden Katalysatoren hergestellt. Häufig werden Trägerkatalysatoren verwendet, auf die das katalytisch aktive, metallische Silber mittels eines geeigneten Verfahrens aufgebracht wurde. Als Trägermaterial können grundsätzlich verschiedene poröse Materialien, wie z. B. Aktivkohle, Titan-, Zirkonium- oder Siliciumdioxide oder keramische Massen oder Mischungen dieser Materialien, eingesetzt werden. In der Regel wird α-Aluminiumoxid als Trägermaterial verwendet.

Neben Silber und Trägermaterial enthalten diese Katalysatoren zumeist Promotoren zur Verbesserung der katalytischen Eigenschaften. Gemäß Stand der Technik werden beispielsweise Alkali- und/oder Erdalkalimetallverbindungen in geringen Mengen auf den Träger aufgebracht. Einige Schriften lehren die Verwendung von Übergangsmetallen wie Wolfram oder Molybdän. Ein besonders bevorzugter Promotor bei Silberkatalysatoren ist Rhenium. Katalysatoren, die Rhenium und/oder andere Übergangsmetallpromotoren in Kombination mit Alkali- und/oder Erdalkalimetallverbindungen enthalten, werden aufgrund ihrer hohen Selektivität industriell bevorzugt eingesetzt.

Im Laufe der Zeit sind eine Reihe von Verfahren zur Direktoxidation von Alkenen, insbesondere von Ethylen, unter Verwendung verschiedener Silberkatalysatoren entwickelt worden mit dem Ziel, die Selektivität und/oder Aktivität positiv zu beeinflussen. Unter Selektivität ist der molare Prozentsatz an Alkylen, der zu Alkylenoxid reagiert, zu verstehen. Die Aktivität wird durch die Alkylenoxid-Konzentration am Reaktorausgang bei ansonsten konstanten Bedingungen, wie beispielsweise Temperatur, Druck, Gasmenge, Katalysatormenge, charakterisiert. Je höher die Alkylenoxidkonzentration, desto höher ist die Aktivität. Je niedriger die zum Erreichen einer bestimmten Alkylenoxid-Konzentration erforderliche Temperatur ist, desto höher ist die Aktivität. Aufgrund der großen Mengen an beispielsweise Ethylenoxid, die in industriellen Verfahren mit dem Direktoxidationsverfahren produziert werden, kommt jeglicher Erhöhung der Selektivität bzw. Aktivität eines Katalysators eine erhebliche wirtschaftliche Bedeutung zu. Neben der Aktivität ist auch die Lebensdauer des Katalysators von enormer wirtschaftlicher Bedeutung. Die Aktivität und/oder Selektivität der bekannten Katalysatoren nehmen mit fortschreitender Einsatzdauer ab, so dass letztendlich ein wirtschaftlich ungünstiger Katalysatorwechsel erfolgen muss.

Verfahren zur Verbesserung der Aktivität und/oder Selektivität von gebrauchten Silberkatalysatoren beruhen meist darauf, dass ein gebrauchter Katalysator nachbehandelt bzw. regeneriert wird. Die Nachbehandlung besteht beispielsweise darin, dass der gebrauchte Katalysator mit einer Lösung bestehend aus Wasser, einem wassermischbaren Lösungsmittel und einer Cäsium- und/oder Rubidiumverbindung, imprägniert wird. Der derart getränkte Katalysator wird getrocknet und der so getrocknete Katalysator wird dann wieder in der Oxidationsreaktion eingesetzt. Solche Verfahren werden beispielsweise in DE 25 19 599, DE 26 11 856, DE 26 36 680, DE 26 49 359, EP 0 101 008 und DE 29 38 245 beschrieben. Bei den Regenerierungsverfahren, die in der US 4,529,714 bzw. US 4,391,735 beschrieben werden, wird der Silber enthaltende Katalysator in ganz ähnlicher Weise behandelt; allerdings enthält die Regenerierungslösung hier zusätzlich Hydrazin bzw. aliphatische oder aromatische Säuren als Additive.

Die beschriebenen Verfahren haben jedoch den Nachteil, dass der Oxidationsprozess für die gesamte Zeitdauer der Behandlung des Katalysators unterbrochen werden muss. Von Vorteil erscheint ein Verfahren, bei dem Aktivität und/oder Selektivität des Silberkatalysators zu gegebener Zeit während des laufenden Verfahrens in situ durch eine geeignete Behandlung verbessert werden kann. Der Begriff "in situ", wie er im Rahmen der Erfindung verwendet wird, bedeutet, dass eine Behandlung des Katalysators während der laufenden Oxidationsreaktion durchgeführt wird, ohne dass dabei das Oxidationsverfahren unterbrochen wird.

Ein in situ-Regenerationsverfahren wird in der US 6,624,116 beschrieben. Dieses Dokument betrifft ein Verfahren zur Regenerierung eines Katalysators, der in einem autothermalen Oxidationsverfahren zur Umsetzung von paraffinen Kohlenwasserstoffen zu Olefinen verwendet wird. Die Regenerierung wird durch Einleiten einer verdampfbaren Verbindung der Metalle der Gruppe 8b und/oder durch Einleiten eines verdampfbaren Promotors zusammen mit dem Reaktionsgemisch in den Reaktor erreicht. Silberhaltige Katalysatoren und Verfahren zur Direktoxidation von Alkenen zu Alkylenoxiden werden in der US 6,624,116 nicht beschrieben.

Es bestand demnach der Bedarf nach vorteilhaften Verfahren zur Herstellung von Alkylenoxiden durch Direktoxidation von Alkenen an Silber enthaltenden Katalysatoren, bei denen die oben beschriebenen Nachteile, also beispielsweise häufiger Produktionsausfall und/oder Aktivitäts- und/oder Selektivitätsverluste während fortschreitender Einsatzdauer, minimiert werden. Weiterhin bestand der Bedarf nach einem vorteilhaften Regenerierungsverfahren für Silber enthaltende Katalysatoren, die bei der Direktoxidation von Alkenen eingesetzt werden.

Überraschend wurde nun gefunden, dass durch eine in situ-Behandlung eines Silber enthaltenden Katalysators mit einem Ethanol enthaltendem Gemisch, die während der Herstellung eines Alkylenoxids durch Direktoxidation eines Alkens mit Sauerstoff durchgeführt wird, die Selektivität und die Aktivität positiv beeinflusst wird.

Demnach betrifft die vorliegende Erfindung ein kontinuierliches Verfahren zur Herstellung eines Alkylenoxids durch Direktoxidation eines Alkens mit Sauerstoff, umfassend
(i) kontinuierliches Inkontaktbringen eines Silber enthaltenden Katalysators mit einem Gemisch G1, enthaltend das Alken und Sauerstoff, für eine Laufzeit Δt(i);
(ii) zumindest einmaliges Inkontaktbringen des Katalysators gemäß (i) während des kontinuierlichen Inkontaktbringens gemäß (i) mit einem zusätzlichen Gemisch G2, enthaltend Ethanol, für eine Laufzeit Δt(ii),
wobei Δt(i) > Δt(ii) ist.

Das erfindungsgemäße Verfahren kann hierbei auch als ein Regenerierungsverfahren zur Regenerierung eines Silber enthaltenden Katalysators, der bei der Direktoxidation von Alkenen zu Alkylenoxiden eingesetzt wird, verstanden werden, bei dem die Regenerierung des Katalysators in situ, also wie oben definiert, während der laufenden Oxidationsreaktion durchgeführt wird, ohne dass dabei das Oxidationsverfahren unterbrochen wird.

Demnach betrifft die Erfindung die Verwendung des oben genannten Verfahrens zur in situ-Regenerierung eines Silber enthaltenden Katalysators.

Das erfindungsgemäße Verfahren eignet sich besonders für die Direktoxidation von Alkenen mit 2 bis 4 Kohlenstoffatomen, insbesondere zur Direktoxidation von Ethylen oder Propen. Besonders bevorzugt wird das Verfahren zur Direktoxidation von Ethylen zu Ethylenoxid eingesetzt.

Demnach betrifft die vorliegende Erfindung auch ein Verfahren wie oben beschrieben, wobei das Alken Ethylen und das Alkylenoxid Ethylenoxid ist. Ebenso betrifft die Erfindung die Verwendung dieses Verfahrens zur in situ Regenerierung eines Silber enthaltenden Katalysators, während dieser zur Herstellung von Ethylenoxid eingesetzt wird.

Als Katalysatoren können im erfindungsgemäßen Verfahren grundsätzlich alle zur Herstellung von Ethylenoxid aus Ethylen und Sauerstoff geeigneten, Silber enthaltenden Katalysatoren verwendet werden bzw. durch das erfindungsgemäße Verfahren in situ regeneriert werden. Bevorzugt handelt es sich um geträgerte Katalysatoren, d.h. um Katalysatoren, die mindestens ein inertes Trägermaterial enthalten. Als Trägermaterial kann prinzipiell jeder poröse Werkstoff dienen, der unter den Bedingungen der erfindungsgemäßen Direktoxidation stabil ist, beispielsweise Aktivkohle, Aluminiumoxide, Titan-, Zirkonium- oder Siliciumdioxide, Siliciumcarbid oder andere keramische Massen oder geeignete Mischungen dieser Materialien. Bevorzugt enthält der Katalysator Aluminiumoxid, beispielsweise mindestens ein alpha-, gamma- oder theta-Aluminiumoxid, insbesondere ein alpha-Aluminumoxid.

Demnach betrifft die vorliegende Erfindung auch Verfahren, wie oben beschrieben, wobei der Silber enthaltende Katalysator ein inertes Trägermaterial, bevorzugt alpha-Aluminiumoxid, umfasst.

Gemäß einer bevorzugten Ausführungsform wird das Verfahren mit einem Katalysator durchgeführt, der als Trägermaterial ein alpha-Aluminiumoxid mit einer BET-Oberfläche, bestimmt nach Brunauer et al., J. Am. Chem. Soc. 60, S. 309 (1938), von 0,1 bis 20 m²/g, bevorzugt 0,2 bis 10 m²/g, weiter bevorzugt im Bereich von 0,3 bis 5 m²/g, weiter bevorzugt im Bereich von 0,4 bis 3 m²/g, weiter bevorzugt im Bereich von 0,5 bis 2 m²/g und besonders bevorzugt im Bereich von 0,7 bis 1,2 m²/g enthält. Weiterhin weist dieses alpha-Aluminiumoxid bevorzugt Porenvolumen im Bereich von 0,1 bis 2,0 ml/g, bevorzugt im Bereich von 0,2 bis 1,2 ml/g, weiter bevorzugt im Bereich von 0,4 bis 1,0 ml/g und besonders bevorzugt im Bereich von 0,4 bis 0,8 ml/g, gemessen mit dem Verfahren der Quecksilberporosimetrie gemäß DIN 66133, auf und zeigt eine Kaltwasseraufnahme bei 20 °C innerhalb von 5 min von 0,1 bis 2,0 ml/g, bevorzugt von 0,2 bis 1,2 ml/g, weiter bevorzugt von 0,4 bis 1,0 ml/g, und besonders bevorzugt im Bereich von 0,4 bis 0,8 ml/g.

Gemäß einer weiter bevorzugten Ausführungsform hat das alpha-Aluminiumoxid eine Reinheit von größer 75 %, bevorzugt eine Reinheit von größer 80 %, weiter bevorzugt eine Reinheit von größer 85 %, weiter bevorzugt eine Reinheit von größer 90 %, und ganz besonders bevorzugt eine Reinheit von größer 98 %.

Der Begriff "alpha-Aluminiumoxid" umfasst hierbei auch alpha-Aluminiumoxide, die weitere Bestandteile enthalten, beispielsweise eine oder mehrere Verbindungen, bevorzugt Oxide der Elemente ausgewählt aus der Gruppe bestehend aus Natrium, Kalium, Rubidium, Cäsium, Magnesium, Calcium, Strontium, Barium, Silicium, Eisen, Zirkonium, und Gemischen aus einem oder mehreren dieser Elemente. Beispielsweise enthält das alpha-Aluminiumoxid ein oder mehrere Verbindungen ausgewählt aus der Gruppe bestehend aus Siliciumdioxid, Natriumoxid, Eisen-(III)-oxid, Titandioxid, Calciumoxid, Zirkoniumoxid, Kaliumoxid und Magnesiumoxid.

Bevorzugt ist das inerte Trägermaterial ein alpha-Aluminiumoxid in einer Reinheit von mindestens 98 %.

Gemäß einer weiter bevorzugten Ausführungsform umfasst das alpha-Aluminiumoxid, welches bevorzugt eine Reinheit von mindestens 98 % aufweist, weniger als 1 Gew.-% Siliciumdioxid. Umfasst das alpha-Aluminiumoxid Natriumoxid, so umfasst es dies bevorzugt einer Menge von 0,01 bis 0,5 Gew.-%, bevorzugt in einer Menge von 0,08 bis 0,12 Gew.-%, jeweils bezogen auf das Gesamtgewicht des inerten Trägermaterials. Umfasst das alpha-Aluminiumoxid Eisen-(III)-oxid, so umfasst es dies ebenfalls bevorzugt einer Menge von 0,01 bis 0,5 Gew.-%, bevorzugt in einer Menge von 0,08 bis 0,12 Gew.-%, jeweils bezogen auf das Gesamtgewicht des inerten Trägermaterials. Umfasst das alpha-Aluminiumoxid Titandioxid, so umfasst es dies bevorzugt einer Menge von weniger als 0,5 Gew.-%, bevorzugt in einer Menge von weniger als 0,3 Gew.-%, weiter bevorzugt in einer Menge von weniger als 0,02 Gew.-%, jeweils bezogen auf das Gesamtgewicht des inerten Trägermaterials. Umfasst das alpha-Aluminiumoxid beispielsweise Natriumoxid, so umfasst es dies bevorzugt einer Menge von 0.01 bis 0,5 Gew.-%, bevorzugt in einer Menge von 0,08 bis 0,12 Gew.-%, jeweils bezogen auf das Gesamtgewicht des inerten Trägermaterials. Umfasst das alpha-Aluminiumoxid Calciumoxid, so umfasst es dies bevorzugt einer Menge von 0.01 bis 0,5 Gew.-%, bevorzugt in einer Menge von 0,08 bis 0,12 Gew.-%, jeweils bezogen auf das Gesamtgewicht des inerten Trägermaterials. Umfasst das alpha-Aluminiumoxid Magnesiumoxid, so umfasst es dies bevorzugt einer Menge von 0,01 bis 0,5 Gew.-%, bevorzugt in einer Menge von 0,08 bis 0,12 Gew.-%, jeweils bezogen auf das Gesamtgewicht des inerten Trägermaterials. Umfasst das alpha-Aluminiumoxid Kaliumoxid, so umfasst es dies bevorzugt einer Menge von 0,01 bis 0,5 Gew.-%, bevorzugt in einer Menge von 0,08 bis 0,12 Gew.-%, jeweils bezogen auf das Gesamtgewicht des inerten Trägermaterials. Üblicherweise enthält das inerte Trägermaterial Zirkoniumdioxid in einer Menge von weniger 0,1 Gew.-%, bevorzugt von weniger als 0,05 Gew.-%, jeweils bezogen auf das Gesamtgewicht des inerten Trägermaterials. Es können beispielsweise Träger des Typs SA5-61 der Fa. Saint-Gobain NorPro oder des Typs 19/30 der Fa. CeramTec verwendet werden.

Die geometrische Form der Trägerpartikel ist grundsätzlich beliebig. Zweckmäßigerweise haben die Trägerpartikel Formen, die eine ungehinderte Diffusion der Reaktionsgase zu der mit den katalytisch aktiven Silberpartikeln belegten äußeren und inneren Oberfläche der Trägerpartikel ermöglicht. Bevorzugte Trägerformen sind Tabletten, Stränge, Kugeln, Linsen- oder eiförmige Formkörper, besonders bevorzugt Stränge. Ganz besonders bevorzugt sind Hohlstränge, gelochte Sternstränge oder gelochte Lobalstränge.

Was die Menge an Silber betrifft, so werden im erfindungsgemäßen Verfahren bevorzugt Katalysatoren eingesetzt, die Silber in einer Menge im Bereich von 10 bis 30 Gew.-% enthalten. Besonders bevorzugt sind Katalysatoren, die Silber in einer Menge im Bereich von 10 bis 20 Gew.-%, weiter bevorzugt von 12 bis 18 Gew.-% und besonders bevorzugt von 13 bis 16 Gew.-%, jeweils bezogen auf das Gesamtgewicht des Katalysators, enthalten.

Demnach betrifft die vorliegende Erfindung auch ein Verfahren, wie oben beschrieben, wobei der Katalysator Silber in einer Menge von 10 bis 30 Gew.-% bezogen auf das Gesamtgewicht des Katalysators enthält.

Neben Silber enthalten die im erfindungsgemäßen Verfahren eingesetzten Katalysatoren bevorzugt mindestens einen Promotor, beispielsweise sechs, fünf, vier, drei, zwei Promotoren oder 1 Promotor. Unter einem "Promotor" wird im Rahmen der Erfindung ein Bestandteil des Katalysators verstanden, durch den, im Vergleich zu einem Katalysator, der den Bestandteil nicht enthält, eine Verbesserung in einer oder mehreren katalytischen Eigenschaften, zum Beispiel Selektivität, Aktivität, Umsatz, und/oder Ausbeute bzw. Raum-Zeit-Ausbeute erreicht wird. Es sind solche Verbindungen bevorzugt, die unter den Reaktionsbedingungen chemisch weitestgehend stabil sind und keine unerwünschten Reaktionen katalysieren. Beispielhaft für mit Promotoren dotierte Silberkatalysatoren, die im erfindungsgemäßen Verfahren eingesetzt werden können, seien die Silberkatalysatoren, die in der DE-A 23 00 512 beispielsweise auf Seite 4, Zeile 5 bis Seite 11, Zeile 5, in der DE-A 25 21 906 beispielsweise auf Seite 2, Zeile 12 bis Seite 7, in der EP-A 0 014 457 beispielsweise auf Seite 4 Zeile 14 bis Seite 6 Zeilen 30, in der DE-A 24 54 972 beispielsweise auf Seite 3, 3. Abschnitt und auf Seite 5 bis Seite 14, in der EP-A 0 357 293 beispielsweise Spalte 1, Zeilen 10 bis Zeile 24 und in den Spalten 4 und 5, EP-A 0 011 356 beispielsweise auf Seite 1, Zeile 19 bis Seite 2, Zeile 14 und auf den Seiten 3 bis Seite 5, Zeile 20, sowie in der EP-A 0 266 015, der EP-A 0 172 565, der EP A 0 085 237 und der DE-A 25 60 684 beschrieben sind, genannt.

Demnach betrifft die vorliegende Erfindung auch ein Verfahren, wie oben beschrieben, wobei der Katalysator zusätzlich mindestens einen Promotor umfasst. Weiterhin betrifft die Erfindung die Verwendung dieses Verfahrens zur in situ-Regenerierung eines Silber und mindestens einen Promotor enthaltenden Katalysators, während dieser zur Herstellung eines Alkylenoxids eingesetzt wird.

Als Promotoren seien insbesondere Alkalimetall- und Erdalkalimetallhydroxide oder -salze sowie die Verbindungen der Elemente der 6. und 7. Nebengruppe des Periodensystems, insbesondere Verbindungen der Elemente Wolfram, Molybdän und/oder Rhenium genannt. Besonders geeignet für das Verfahren sind Katalysatoren, die einen Promotor ausgewählt aus der Gruppe bestehend Re, W, Mo, Rb, Li, K, Cs, Sr, Ba, Ca, S, P, B, In, Sn, Sb, TI, Pb, und Bi und Mischungen aus zwei oder mehr davon enthalten.

Demnach betrifft die vorliegende Erfindung auch ein Verfahren, wie oben beschrieben, wobei der mindestens eine Promotor ausgewählt ist aus der Gruppe bestehend aus Re, W, Mo, Rb, Li, K, Cs, Sr, Ba, Ca, S, P, B, In, Sn, Sb, TI, Pb, und Bi und Mischungen aus zwei oder mehr davon.

Ganz besonders geeignet ist das Verfahren für Katalysatoren, die mindestens einen Promotor ausgewählt aus der Gruppe bestehend aus Li, Cs, W, Mo, S und Re und Mischungen aus zwei oder mehr davon enthalten. Gemäß einer besonders bevorzugten Ausführungsform enthält der Katalysator als Promotor mindestens Lithium und Schwefel.

Die im erfindungsgemäßen Verfahren eingesetzten Katalysatoren werden bevorzugt durch Aufbringen der aktiven Bestandteile auf das inerte Trägermaterial, d.h. durch Aufbringen von Silber und Aufbringen des mindestens einen Promotors, hergestellt. Hierzu können grundsätzlich alle Tränk- und Abscheideverfahren des Standes der Technik verwendet werden, wobei diese Verfahren eine oder mehrere Tränk- bzw. Abscheidestufen sowie eine oder mehrere Calcinierungsstufen umfassen können. Beispielhaft seien die Herstellverfahren für Silberkatalysatoren genannt, wie sie in DE-A 23 00 512 beispielsweise auf Seite 11, Zeile 14 bis Seite 21, Zeile 4 und in den Beispielen, in der DE-A 25 21 906 beispielsweise auf Seite 7 bis Seite 14, Zeile 4, in der EPA 0 014 457 beispielsweise auf Seite 4 Zeile 1 bis Seite 8 Zeile 16 und in Beispiel 1 und 2, EP-A 0 011 356 beispielsweise auf Seite 2, Zeile 15 bis Seite 6, Zeile 13, in der DE-A 24 54 972 beispielsweise auf Seite 3, 3. Abschnitt und auf Seite 5 bis Seite 14, in der EP-A 0 357 293 beispielsweise in Spalte 1, Zeile 25 bis Spalte 4, Zeile 17, sowie in der EP-A 0 384 312, der DE-A 33 21 895, EP-A 0 229 465, der DE-A-31 50 205, und der EP-A 0 172 565 offenbart sind. Bei der Herstellung des Katalysators wird Silber bevorzugt in Form einer Silberverbindung, die ein Salz oder ein Silberkomplex sein kann, auf dem oben beschriebenen inerten Trägermaterial aufgebracht. Bevorzugt wird die Silberverbindung gelöst, insbesondere gelöst in Wasser, aufgebracht. Um die Silberverbindung in löslicher Form zu erhalten, kann der Silberverbindung, wie beispielsweise Silber I-Oxid oder Silber I-Oxalat, weiterhin in geeigneter Weise ein Komplexierungsmittel, wie Ethanolamin, Oxalsäure, und/oder Ethylendiamin zugesetzt werden, das gleichzeitig auch als Reduktionsmittel wirken kann. Besonders bevorzugt wird Silber in Form einer Silber-Amin-Verbindung, bevorzugt einer Silber-Ethylendiamin-Verbindung, aufgebracht. Die Promotoren werden weiterhin bevorzugt in Form ihrer Salze, beispielsweise der Halogenide, insbesondere der Fluoride, Chloride, Carboxylate, Nitrate, der Sulfate oder Sulfide, Phosphate, Cyanide, Hydroxide, Carbonate oder der Salze von Heteropolysäuren, insbesondere von Heteropolysäuren der Elemente der 6. und 7. Nebengruppe des Periodensystems, besonders bevorzugt in Form der Salze von Heteropolysäuren des Wolframs, Molybdäns und/oder Rheniums, im Abscheide- bzw. Tränkverfahren eingesetzt.

Als im erfindungsgemäßen Verfahren bevorzugt eingesetzte Katalysatoren, seien beispielhaft Silberkatalysatoren mit einem Silbergehalt von 10 bis 30 Gew.-%, bezogen auf das Gesamtgewicht des Katalysators, einem Gehalt an Lithium und/oder Cäsium von 1 bis 5000 Gew.-ppm, einem Gehalt an Wolfram und/oder Molybdän von 1 bis 5000 Gew.-ppm und einem Gehalt an Schwefel von 1 bis 500 Gew.-ppm jeweils bezogen auf das Gesamtgewicht des Katalysator, genannt. Weiterhin enthält der Katalysator optional Rhenium in einem Gehalt von 0 bis 3 000 Gew.-ppm, bezogen auf das Gesamtgewicht des Katalysators.

Das Inkontaktbringen der oben beschriebenen Katalysatoren mit Gemisch G1 gemäß (i) kann grundsätzlich unter jeglichen für die Direktoxidation geeigneten Bedingungen durchgeführt werden.

Typischerweise erfolgt das Inkontaktbringen in mindestens einem Reaktor, wobei die in den Ethylenoxidherstellungsverfahren des Standes der Technik üblichen Reaktoren eingesetzt werden können. Es können beispielsweise außengekühlte Rohrbündelreaktoren (vgl. Ullmann's Encyclopedia of Industrial Chemistry; 5th ed.; vol. A10; S. 117-135, 123-125; VCH Verlagsgesellschaft; Weinheim 1987) oder auch Reaktoren mit loser Katalysatorschüttung und Kühlrohren, beispielsweise die in DE-A 34 14 717, EP-A 0 082 609 und EP-A 0 339 748 beschriebenen Reaktoren, eingesetzt werden. Bevorzugt wird das Inkontaktbringen gemäß (ii) in mindestens einem Rohrreaktor, bevorzugt in einem Rohrbündelreaktor durchgeführt. Das Gemisch G1 wird hierbei bevorzugt gasförmig in den Reaktor eingespeist und dort mit dem Katalysator in Kontakt gebracht.

Das eingespeiste, Alken, bevorzugt Ethylen, und Sauerstoff enthaltende Gemisch G1 enthält bevorzugt eine Alken-Menge im Bereich von 10 bis 80 Vol.-%, bevorzugt von 20 bis 60 Vol.-%, weiter bevorzugt von 25 bis 50 Vol.-%, und ganz besonders bevorzugt von 30 bis 40 Vol.-%, bezogen auf das Gesamtvolumen von G1. Der Sauerstoffgehalt des Gemisches G1 liegt zweckmäßigerweise in einem Bereich, in dem keine explosionsfähigen Gasgemische vorliegen. Bevorzugt enthält G1 eine SauerstoffMenge im von höchstens 10 Vol.-%, bevorzugt eine Menge von höchstens 9 Vol.-%, weiter bevorzugt eine Menge von höchstens 8 Vol.-%, und ganz besonders bevorzugt eine Menge von höchstens 7 Vol.-%, bezogen auf das Gesamtvolumen von G1.

Demnach beschreibt die vorliegende Erfindung auch ein Verfahren, wie oben ausgeführt, wobei das Gemisch G1 das Alken, bevorzugt Ethylen, in einer Menge im Bereich von 30 bis 40 Vol.-% und Sauerstoff in einer Menge von höchstens 7 Vol.-% enthält.

Neben diesen Bestandteilen umfasst das Gemisch G1 bevorzugt noch weiter Bestandteile. Gemäß einer bevorzugten Ausführungsform betrifft die vorliegende Erfindung ein Verfahren wie oben beschrieben, wobei das Gemisch G1 ein Inertgas enthält. Unter Inertgas sind solche Gase zu verstehen, die sich unter den Reaktionsbedingungen der vorliegenden Erfindung weitestgehend inert verhalten. Insbesondere ist das Inertgas ausgewählt aus der Gruppe bestehend aus Stickstoff, Argon, Methan, Kohlenmonoxid, Helium und Mischungen aus zwei oder mehr davon. Insbesondere umfasst das Gemisch G1 zusätzlich zu Ethylen und Sauerstoff Methan. Enthält das Gemisch G1 Methan, enthält es dies bevorzugt in einer Menge im Bereich von 10 bis 80 Vol.-%, bevorzugt von 20 bis 70 Vol.-%, weiter bevorzugt von 30 bis 60 Vol.-%, und ganz besonders bevorzugt von 40 bis 50 Vol.-%, jeweils bezogen auf das Gesamtvolumen von G1.

Weiterhin kann das Gemisch G1 Wasserdampf enthalten. Enthält das Gemisch G1 Wasserdampf, so enthält es diesen bevorzugt in einer Menge im Bereich von 0,05 bis 5,0 Vol.-%, bevorzugt von 0,05 bis 3,0 Vol.-%, weiter bevorzugt von 0,05 bis 2,0 Vol.-%, weiter bevorzugt von 0,05 bis 1,0 Vol.-% und ganz besonders bevorzugt von 0,05 bis 0,5 Vol.-%, jeweils bezogen auf das Gesamtvolumen von G1.

Gemäß einer besonders bevorzugten Ausführungsform enthält das Gemisch G1 Methan in einer Menge von 40 bis 50 Vol.-% und Wasserdampf in einer Menge von 0,05 bis 0,5 Vol.-%, jeweils bezogen auf das Gesamtvolumen von G1.

Als weiteren Bestandteil kann das Gemisch Kohlenstoffdioxid enthalten. Die Kohlenstoffdioxidmenge im Gemisch G1 beträgt im Allgemeinen weniger als 2,5 Vol.-%, bevorzugt weniger als 2 Vol.-%, beispielsweise bevorzugt im Bereich von 0 bis 1,8 Vol.-%, weiter bevorzugt im Bereich von 0 bis 1,5 Vol.-%, weiter bevorzugt im Bereich von 0 bis 1,0 Vol.-%, jeweils bezogen auf das Gesamtvolumen von G1.

Weiterhin kann das Gemisch G1, umfassend Ethylen und Sauerstoff, eine Halogenverbindung enthalten. Bei dem Halogen handelt es sich vorzugsweise um Chlor, während bevorzugte Halogenverbindungen organische Halogenverbindungen sind, bevorzugt Halogenide von Kohlenwasserstoffen mit 1 bis 10 Kohlenstoffatomen. Geeignet sind beispielsweise chlorierte aromatische Verbindungen, z.B. Chlorbenzol, Dichlorbenzole oder chlorierte Toluole. Besonders bevorzugt ist die Halogenverbindung ausgewählt aus der Gruppe bestehend aus Ethylchlorid, 1,1-Dichloroethan, 1,2-Dichloroethan, Methylchlorid, Methylenchlorid, Vinylchlorid und Mischungen aus ein oder mehr dieser Verbindungen.

Demnach betrifft die vorliegende Erfindung auch ein Verfahren, wie oben beschrieben, wobei das Gemisch G1 zusätzlich ein organisches Halogenid, bevorzugt ein organisches Halogenid ausgewählt aus der Gruppe bestehend aus Ethylchlorid, Methylchlorid, Methylenchlorid, Vinylchlorid, 1,1-Dichloroethan und 1,2-Dichloroethan oder Mischungen aus ein oder mehr dieser Verbindungen enthält. Ganz besonders bevorzugt enthält das Gemisch G1 Ethylchlorid.

Die Halogenverbindung, bevorzugt das organische Halogenid, wird im Allgemeinen in einer Konzentration von 0,1 bis 2000 Vol.-ppm, bevorzugt in einer Konzentration von 1 bis 1000 Vol.-ppm, weiter bevorzugt in einer Konzentration von 0,5 bis 100 Vol.-ppm, besonders bevorzugt in einer Menge von 1 bis 20 Vol.-ppm jeweils bezogen auf das Gesamtvolumen des Gemisches G1 verwendet. Hierbei kann die Menge an Ethylchlorid im kontinuierlich in den Reaktor eingespeisten Gemisch G1 während der Laufzeit Δt(i) variieren. Je nach Bedarf wird die Menge an Ethylchlorid während der Reaktion angepasst. Üblicherweise wird die Ethylchloridkonzentration zu Beginn der Laufzeit At(i) geringer sein als am Ende der Laufzeit Δt(i), d.h. die Ethylchloridkonzentration wird üblicherweise im Verlauf des Inkontaktbringens erhöht. Dieses Erhöhen kann kontinuierlich oder schrittweise erfolgen, wobei üblicherweise eine schrittweise Erhöhung der Ethylchloridkonzentration erfolgt. Üblicherweise wird zu Beginn der Laufzeit Δt(1) eine Menge an Ethylchlorid gewählt, die im Bereich von 1 bis 4 Vol.-ppm liegt, während die Menge an Ethylchlorid am Ende der Laufzeit Δt(i) üblicherweise im Bereich von 4 bis 20 Vol.-ppm liegt, jeweils bezogen auf das Gesamtvolumen von G1.

Neben den genannten Bestandteilen kann das Gemisch G1 weiterhin eine geeignete Stickstoffverbindung, beispielsweise Stickstoffmonoxid, Stickstoffdioxid, N₂O₄, Ammoniak, Nitromethan, Nitroethan und/oder N₂O₃ enthalten, wobei Stickstoffmonoxid und/oder Stickstoffdioxid besonders vorteilhaft sind. Die Stickstoffverbindung wird im Allgemeinen in Konzentrationen von 0,1 bis etwa 2000 Vol.-ppm, bevorzugt im Bereich von 0,1 bis 2000 Vol.-ppm, weiter bevorzugt im Bereich von 1 bis 1000 Vol.-ppm und besonders bevorzugt im Bereich von 50 bis 500 Vol.-ppm, jeweils bezogen auf Gesamtvolumen von G1 verwendet.

Demnach betrifft die vorliegende Erfindung auch ein Verfahren, wie oben beschrieben, wobei das Gemisch G1 zusätzlich eine Stickstoffverbindung, bevorzugt Stickstoffmonoxid und/oder Stickstoffdioxid enthält.

Gemäß einer besonders bevorzugten Ausführungsform enthält das Gemisch G1 Ethylen in einer Menge im Bereich von 30 bis 40 Vol.-%, Sauerstoff in einer Menge von höchstens 78 Vol.-%, Wasserdampf in einer Menge im Bereich von 0,05 bis 0,5 Vol.-%, Kohlendioxid in einer Menge im Bereich von 0,1 bis 1,0 Vol.-%, Methan in einer Menge im Bereich von 40 bis 50 Vol.-%, sowie Ethylchlorid in einer Menge von 1 bis 20 Vol.-ppm, jeweils bezogen auf das Gesamtvolumen des Gemisches G1.

Die oben beschriebenen Bestandteile des Gasgemisches können gegebenenfalls jeweils geringe Mengen an Verunreinigungen aufweisen. Ethylen kann beispielsweise in jeglichen Reinheitsstufen, die für die erfindungsgemäße Gasphasenoxidation geeignet sind, verwendet werden. Geeignete Reinheitsstufen schließen ein, sind aber nicht limitiert auf, "Polymer Grade" Ethylen, welches typischerweise ein Reinheit von mindestens 99 % hat und "Chemical Grade" Ethylen, welches eine geringere Reinheit von typischerweise größer 95 % aufweist. Die Verunreinigungen bestehen hierbei typischerweise vor allem aus Ethan, Propan und/oder Propen

Im Rahmen der Erfindung kann das Ethylen auf jegliche geeignet Art und Weise bereitgestellt werden. Im Allgemeinen stammt das im erfindungsgemäßen Verfahren eingesetzte Ethylen aus Steam-Cracking-Verfahren, beispielsweise Steam-Cracking von Öl bzw. Naphta oder Steam-Cracking von Ethan, welches als Begleitgas bei der Erdöl- oder Erdgasförderung anfällt. Ebenso kann das Ethylen auch aus einer katalytischen, oxidativen oder autothermen Dehydrierung von Ethan stammen.

Gemäß einer alternativen Ausführungsform der Erfindung wird Ethylen eingesetzt, welches durch Dehydratisierung von Ethanol gewonnen wird. Ethanol wiederum kann sehr leicht aus zucker- oder stärkehaltigen Pflanzen wie Zuckerrohr, Zuckerrüben, Mais, Roggen usw. durch Fermentation gewonnen werden. Aufgrund der guten Verfügbarkeit dieser nachwachsenden Rohstoffe ist langfristig eine kostengünstige Versorgung mit Ethanol und daraus durch katalytische Dehydratisierung hergestelltem Ethylen gesichert.

Demnach betrifft die vorliegende Erfindung auch ein Verfahren wie oben beschrieben, zusätzlich umfassend
(a) Bereitstellen von Ethylen durch Dehydratisierung von Ethanol.

Die Dehydratisierung von Ethanol zu Ethylen ist eine an sich bekannte Technologie und kann wie in der nachstehend aufgeführten Literatur beschrieben durchgeführt werden. Bis in die 60er Jahre war die Ethylen-Produktion aus Ethanol in kleinem Maßstab (2 - 10 kt/a) recht verbreitet, siehe Chem. Eng. 1967, 100-102. An Orten ohne Zugang zu Kohlenwasserstoffen und mit günstigem Ethanol aus Biomasse (insbesondere Zuckerrohr) wurde die Ethanol-Dehydratisierung weiterbetrieben, siehe Chem. Eng. 1981, 17. Temperatur-Kontrolle ist entscheidend für die Ethylen-Selektivität. Bei zu niedrigen Temperaturen (< 300 °C) wird Diethylether gebildet, bei zu hohen Temperaturen (> 400 °C) erhöht sich der Anteil an Acetaldehyd. Für die in US 4,134,926 beschriebene großtechnische Synthese von Ethylen in einem Wirbelschicht-Reaktor bei Temperaturen zwischen 400 und 480 °C an einem SiO₂/Al₂O₃ Crack-Katalysator werden Ausbeuten von 99 % und mehr angegeben. Als Katalysatoren werden allgemein oxidische Katalysatoren, z. B. Al2O3, ZrO2 (siehe Bull. Soc. Chem. Jpn. 1975, 48, 3377), Salze wie Sulfate (siehe J. Catal. 1971, 22, 23), Phosphate (siehe Kinet. Katal. 1964, 5, 347), (Hetero)polyphosphorsäuren (siehe Chem. Lett. 1981, 391.; Ind. Eng. Chem., Prod. Res. Dev. 1981, 20, 734), Zeolithe wie ZSM-5 (siehe J. Catal. 1978, 53, 40), lonenaustauscher-Harze oder auch geträgerte Phosphorsäure genutzt. T.S.R.P. Rao und G.M. Dhar berichten für einen gamma-Al₂O₃ Katalysator bei 350 °C, 375 °C und 400 °C sowohl 100 % Aktivität als auch 100 % Selektivität (Recent Advances in Basic and Applied Aspects of Industrial Catalysis, Studies in Surface Science and Catalysis 1998, Vol. 113, 241).

Die Dehydratisierung von Ethanol kann prinzipiell nach allen im Stand der Technik beschriebenen Verfahren erfolgen. Bevorzugt erfolgt die Dehydratisierung an einem Katalysator umfassend einen Zeolithen des Typs ZSM-5 oder an einem gamma-Aluminiumoxid umfassenden Katalysator. Bevorzugt wird die Dehydratisierung in einem Temperaturbereich von 300 °C bis 400 °C bei Normaldruck durchgeführt. Das Eingangsgas besteht dabei weiter bevorzugt aus gasförmigem Ethanol. Gemäß einer alternativen Ausführungsform wird ein Eingangsgas bestehend aus einem Gemisch aus Ethanol und mindestens einem Inertgas, bevorzugt einem Gemisch aus Stickstoff und Ethanol, eingesetzt. "Bestehen aus Ethanol" bedeutet in diesem Zusammenhang, dass das Ethanol die üblichen in Ethanol enthaltenden Verunreinigungen, wie beispielsweise Methanol, Propanol, Acetaldehyd und/oder Essigsäure enthalten kann. "Bestehen aus einem Gemisch aus Ethanol und mindestens einem Inertgas" bedeutet weiterhin, dass das Gemisch die üblichen in Ethanol enthaltenden Verunreinigungen und/oder die üblichen im verwendeten mindestens einen Inertgas enthaltenden Verunreinigungen, beispielsweise die üblichen im Stickstoff enthaltenden Verunreinigungen, enthalten kann.

Gemäß einer bevorzugten Ausführungsform umfasst das Bereitstellen gemäß (a) eine Reinigung des aus der Dehydratisierung gewonnenen Ethylen enthaltenden Gemisches. Bevorzugt erfolgt die Reinigung durch Gaswäsche und oder Kondensation, wobei Reste von Ethanol absorbiert bzw. auskondensiert werden.

Bevorzugt wird zumindest ein Teil des aus der Dehydratisierung gewonnenen, und gegebenenfalls gereinigten, Ethylens als Bestandteil des Gemisches G1 gemäß (i) eingesetzt.

Demnach betrifft die vorliegende Erfindung auch ein Verfahren wie oben beschrieben, zusätzlich umfassend (a) Bereitstellen von Ethylen durch Dehydratisierung von Ethanol, wobei zumindest ein Teil des Ethylens aus (a) als Bestandteil des Gemisches G1 gemäß (i) eingesetzt wird.

Das Inkontaktbringen des Gemisches G1 mit dem Silber enthaltenden Katalysator wird üblicherweise bei erhöhter Temperatur durchgeführt. Bevorzugt sind Temperaturen im Bereich von 180 °C bis 300 °C, weiter bevorzugt Temperaturen im Bereich von 185 °C bis 290 °C, weiter bevorzugt Temperaturen im Bereich von 190 °C bis 280 °C, und besonders bevorzugt Temperaturen im Bereich von 200 °C bis 270 °C. Demnach betrifft die vorliegende Erfindung auch ein Verfahren, wie oben beschrieben, wobei das Inkontaktbringen gemäß (i) bei einer Temperatur im Bereich von 180 °C bis 300 °C, bevorzugt im Bereich von 200 °C bis 270 °C, erfolgt. Üblicherweise wird (i) bei Drücken im Bereich von 5 bar bis 30 bar durchgeführt. Bevorzugt erfolgt das Inkontaktbringen gemäß (i) bei einem Druck im Bereich von 5 bar bis 25 bar, bevorzugt bei einem Druck im Bereich 10 bar bis 20 bar und besonders bevorzugt bei einem Druck im Bereich von 14 bar bis 20 bar. Demnach betrifft die vorliegende Erfindung auch ein Verfahren, wie oben beschrieben, wobei das Inkontaktbringen gemäß (i) bei einem Druck im Bereich von 14 bar bis 20 bar erfolgt.

Die in (i) angewendete GHSV (gas hourly space velocity) ist abhängig von der Art des gewählten Reaktors, beispielsweise von der Größe/Durchschnittsfläche des Reaktors, und der Form und Größe des Katalysators. Bevorzugt liegt die GHSV im Bereich von 800 bis 10000 pro Stunde, bevorzugt im Bereich von 2000 bis 6000, weiter bevorzugt im Bereich von 3000 bis 6000, wobei sich die Angabe auf das Volumen des Katalysators beziehen.

Demnach betrifft die vorliegende Erfindung auch ein Verfahren, wie oben beschrieben, wobei das Inkontaktbringen gemäß (i) mit einer GHSV im Bereich von 3000 bis 6000 erfolgt.

Typischerweise wird das Inkontaktbringen gemäß (i) für eine Laufzeit Δt(i), die mehr als 100 h beträgt durchgeführt. Unter der Laufzeit Δt(i) wird hierbei die gesamte Laufzeit eines mit einem Katalysator durchgeführten Oxidationsprozesses verstanden, bevor ein Austausch notwendig wird.

Bevorzugt beträgt die Laufzeit Δt(i) mehr als 100 h, bevorzugt mehr als 200 h, weiter bevorzugt mehr als 500 h, und besonders bevorzugt mehr als 1000 h.

Demnach betrifft die vorliegende Erfindung auch ein Verfahren, wie oben beschrieben, wobei die Laufzeit Δt(i) mindestens 100 h beträgt.

Während des Inkontaktbringens gemäß (i) wird der Katalysator, wie oben beschrieben, zusätzlich zumindest einmalig mit einem Gemisch G2 enthaltend Ethanol für eine Laufzeit Δt(ii) in Kontakt gebracht. Dieses Inkontaktbringen erfolgt bevorzugt zu einem Zeitpunkt, an dem bereits eine Deaktivierung oder teilweise Deaktivierung des Katalysators eingesetzt hat.

Das Inkontaktbringen mit dem Ethanol enthaltenden Gemisch gemäß (ii) wird im erfindungsgemäßen Verfahren nicht während der gesamte Laufzeit Δt(i) sondern nur für einen oder mehrere begrenzte Zeiträume Δt(ii) während der Laufzeit Δt(i) durchgeführt. Bevorzugt ist die Laufzeit Δt(ii) kleiner als 24 h. Weiter bevorzugt liegt die Laufzeit Δt(ii) im Bereich von 0,1 bis 12 h, bevorzugt im Bereich von 0,1 bis 6 h, weiter bevorzugt im Bereich von 0,1 bis 5 h, und besonders bevorzugt im Bereich von 0,1 bis 4 h.

Demnach betrifft die vorliegende Erfindung auch ein Verfahren, wie oben beschrieben, wobei die Laufzeit Δt(ii) im Bereich von 0,1 bis 4 h liegt.

Je nach Bedarf kann während der Laufzeit Δt(i) das Inkontaktbringen gemäß (ii) einmal oder mehrere Male durchgeführt werden, beispielsweise fünfmal, viermal, dreimal, zweimal oder einmal. Wie oft Schritt (ii) durchgeführt wird, ist beispielsweise abhängig vom Grad der Deaktivierung des Katalysators oder von der Länge der Laufzeit Δt(i). Im Falle, dass das Inkontaktbringen gemäß (ii) nicht nur einmal sondern mehrmals durchgeführt wird, ist die Summe aller Laufzeiten Δt(ii) ebenfalls kleiner als Δt(i). Bevorzugt ist das Verhältnis von Δt(i) zur Summe der Laufzeiten Δt(ii), also zu Σ(Δt(ii)), im Bereich von 10 bis 10000, beispielsweise im Bereich von 100 bis 10000, bevorzugt im Bereich von 10 bis 1000.

Zudem kann die Laufzeit Δt(ii) beim mehrmaligen Inkontaktbringen je nach Bedarf im oben genannten Rahmen variieren d.h. sollte der Katalysator mehr als einmal mit dem Gemisch G2 jeweils für eine Laufzeit Δt(ii) in Kontakt gebracht werden, kann Δt(ii) von Mal zu Mal innerhalb der oben genannten Bereiche variiert werden.

Zum Inkontaktbringen gemäß (ii) wird das Gemisch G2 bevorzugt zusammen mit dem Gemisch G1 in den mindestens einen Reaktor eingespeist und dort mit dem Katalysator, wie oben beschrieben, in Kontakt gebracht. Hierbei können G1 und G2 an verschiedenen Stellen oder zusammen an derselben Position in den mindestens einen Reaktor eingespeist werden. Bevorzugt wird das Gemisch G2 vor Einspeisung mit G1 vermischt, d.h. bevorzugt zum Gemisch G1 zudosiert, und das entstehende Gemisch aus G1 und G2 wird anschließend als Eingangsgas in den mindestens einen Reaktor geleitet.

Demnach betrifft die vorliegende Erfindung auch ein Verfahren, wie oben beschrieben, wobei das Gemisch G2 vor dem Inkontaktbringen mit dem Katalysator gemäß (ii) zum Gemisch G1 zudosiert wird.

Das Inkontaktbringen gemäß (ii) für die Laufzeit Δt(ii) wird hier im Wesentlichen bei denselben Bedingungen durchgeführt, wie das Inkontaktbringen gemäß (i), d.h. bei Temperaturen im Bereich von 180 °C bis 300 °C, bevorzugt bei Temperaturen im Bereich von 185 °C bis 290 °C, weiter bevorzugt Temperaturen im Bereich von 190 °C bis 280 °C, und besonders bevorzugt Temperaturen im Bereich von 200 °C bis 270 °C, durchgeführt. Bevorzugt wird das Gemisch G2 vor dem Zudosieren zu G1 auf eine Temperatur im Bereich von 170 bis 270 °C gebracht wird.

Weiter bevorzugt erfolgt das Inkontaktbringen bei einem Druck im Bereich von 5 bar bis 30 bar, bevorzugt bei einem Druck im Bereich 10 bar bis 20 bar und besonders bevorzugt bei einem Druck im Bereich von 14 bar bis 20 bar. Weiterhin wird das Inkontaktbringen gemäß (ii) für die Laufzeit Δt(ii) im Wesentlichen mit derselben GHSV wie das Inkontaktbringen gemäß (i), d.h. also einer GHSV im Bereich von 800 bis 10000 pro Stunde, bevorzugt im Bereich von 2000 bis 6000, weiter bevorzugt im Bereich von 3000 bis 6000 durchgeführt.

Was die Zusammensetzung des Gemisches G2 betrifft, so enthält dieses Gemisch Ethanol bevorzugt in einer Menge von mindestens 0,01 % bezogen auf das Gesamtgewicht von G2. Bevorzugt besteht das Gemisch aus Ethanol. "Bestehen aus Ethanol" bedeutet in diesem Zusammenhang, dass G2 Ethanol zuzüglich der üblichen in Ethanol enthaltenden Verunreinigungen, wie beispielsweise Methanol, Propanol, Acetaldehyd und/oder Essigsäure enthält. Bei dem in (ii) eingesetzten Ethanol handelt es sich gemäß einer Ausführungsform der Erfindung, um bei der Dehydratisierung von Ethanol gemäß (a) nicht umgesetztes und auf geeignete Weise aufgereinigtes Ethanol.

Gemäß einer alternativen Ausführungsform enthält das Gemisch G2 neben Ethanol mindestens einen verdampfbaren Promotor ausgewählt aus der Gruppe bestehend aus Re, W, und Mo. Der Begriff "verdampfbarer Promotor" wie er im Rahmen der Erfindung verwendet wird umfasst sowohl verdampfbare Verbindungen der Promotoren als auch Gasströme umfassend den Promotor in seiner elementaren Form oder in einer geeigneten, d.h verdampfbaren oder flüchtigen Form.

Handelt es sich bei dem mindestens einen verdampfbaren Promotor um Rhenium, wird das Rhenium bevorzugt als Rheniumverbindung, weiter bevorzugt als ethanolische Lösung einer Rheniumverbindung eingesetzt. Rheniumheptoxid, Ammoniumperrhenat oder Methyltrioxorhenium werden bevorzugt als Rheniumverbindung verwendet.

Demnach betrifft die vorliegende Erfindung auch ein Verfahren, wie oben beschrieben, wobei das Gemisch G2 zusätzlich Rheniumheptoxid enthält.

Im Falle, dass G2 neben Ethanol mindestens einen verdampfbaren Promotor enthält, so enthält G2 den mindestens einen Promotor bevorzugt in einer Menge im Bereich von 10 bis 2000 ppm, bevorzugt in einer Menge im Bereich von 50 bis 500 ppm, bezogen auf das Gesamtgewicht des verwendeten Katalysators.

Das Gemisch G2 wird während der Laufzeit Δt(ii) bevorzugt in eine Menge im Bereich von 0,01 Vol.-% bis 5 Vol.-%, weiter bevorzugt einer Menge im Bereich von 0,01 bis 3 Vol.-%, weiter bevorzugt einer Menge im Bereich von 0,01 bis 2 Vol.-%, weiter bevorzugt einer Menge im Bereich von 0,01 bis 1 Vol.-%, weiter bevorzugt einer Menge im Bereich von 0,01 bis 0,5 Vol.-%, bezogen auf das Gesamtvolumen aus G1 und G2 eingesetzt.

Demnach betrifft die vorliegende Erfindung auch ein Verfahren, wie oben beschrieben, wobei das Gemisch G2 während der Laufzeit Δt(ii) in eine Menge im Bereich von 0,01 bis 0,5 Vol.-%, bezogen auf die Summe der Volumina aus G1 und G2 eingesetzt wird.

Wird das Inkontaktbringen gemäß (ii) mindestens zweimal durchgeführt, können die jeweils durchgeführten Schritte (ii) bei im Wesentlichen gleichen Verfahrensbedingung durchgeführt werden, also beispielsweise mit im Wesentlichen gleicher Zusammensetzung des Gemisches G2, mit im Wesentlichen gleicher Menge von G2, bezogen auf die Summe der Volumina aus G1 und G2, im Wesentlichen gleicher Laufzeit Δt(ii), im Wesentlichen gleicher Temperatur und im Wesentlichen gleichem Druck. Ebenso ist es möglich, dass sich die mindestens zwei Schritte (ii) in ein oder mehr Parametern, wie beispielsweise der Zusammensetzung des Gemisches G2, der eingesetzten Menge des Gemisches G2, der Temperatur, dem Druck und/oder der Laufzeit Δt(ii), unterscheiden.

Im Falle, dass beispielsweise die Zusammensetzung des Gemisches G2, wie oben beschrieben, variiert wird, wird der Katalysator beispielsweise während der Laufzeit Δt(iix) mit einem Gemisch G2 in Kontakt gebracht wird, und während der Laufzeit Δt(iiy) mit einem weiteren Gemisch G2 in Kontakt gebracht, welches sich in seiner Zusammensetzung, d.h. beispielsweise in der Anzahl und/oder Menge der darin gegebenenfalls enthaltenden verdampfbaren Promotoren, vom Gemisch G2, welches während der Laufzeit Δt(iix) eingesetzt wird, unterscheidet.

Gemäß einer Ausführungsform des erfindungsgemäßen Verfahrens wird der Katalysator mindestens einmal mit einem Gemisch G2, welches aus Ethanol besteht, und mindestens einmal mit einem Ethanol und Rhenium enthaltenden Gemisch G2 gemäß (ii) in Kontakt gebracht.

Wird das Inkontaktbringen mindestens zweimal durchgeführt, können die jeweiligen Behandlungen gemäß (ii) zeitlich direkt nacheinander durchgeführt werden. Ebenso ist es möglich, dass die einzelnen Schritte (ii) mit zeitlichen Abständen zueinander erfolgen. Wird das Inkontaktbringen gemäß (ii) mindestens dreimal durchgeführt, können die zeitlichen Abstände zwischen den jeweiligen Schritte (ii) gleich oder unterschiedlich sein. Ebenso ist es möglich zwei oder mehr Schritte (ii) direkt im Anschluss aneinander, d.h. ohne zeitlichen Abstand durchzuführen, und zwei oder mehr Schritte mit zeitlichem Abstand.

Vorteilhafterweise wird das erfindungsgemäße Verfahren in einem Kreisprozess durchgeführt. Dabei wird das Gemisch G1 im Kreislauf durch den Reaktor geleitet und so mit dem Katalysator gemäß (i) in Kontakt gebracht. Zu bestimmten Zeitpunkten wird, wie oben beschrieben, zusätzlich mindestens einmal für einen Zeitraum Δt(ii) G2 zusammen mit G1 durch den Reaktor geleitet. Nach dem Inkontaktbringen wird das durch die Direktoxidation gebildete Ethylenoxid sowie die bei der Umsetzung gebildeten Nebenprodukte aus dem Produktgasstrom entfernt. Beispielsweise wird mindestens ein Teil des im Produktgemisch enthaltenen Kohlenstoffdioxids entfernt. Diese Entfernung erfolgt typischerweise mittels Kohlenstoffdioxid-Absorber. Enthält das Produktgemisch auch Ethanol durch das Inkontaktbringen gemäß (ii) wird dieses gegebenenfalls ebenfalls auf geeignete Weise entfernt. Die Entfernung erfolgt typischerweise durch Gaswäsche und/oder einen Kondensator, wobei das Ethanol absorbiert bzw. auskondensiert wird. Die Abtrennung des Ethylenoxids aus dem Produktgasstrom und seine Aufarbeitung können nach den üblichen Verfahren des Standes der Technik (vgl. Ullmann's Encyclopedia of Industrial Chemistry; 5th Ed.; vol. A2 0; S. 117-135, 123-125; VCH Verlagsgesellschaft; Weinheim 1987) erfolgen. Das nach Abtrennung der Nebenprodukte und von Ethylenoxid bzw. gegebenenfalls von Ethanol erhaltene Gemisch wird nach Ergänzung mit den erforderlichen Mengen an Ethylen, Sauerstoff und optional weiteren Bestandteilen wieder als Gemisch G1, welches die oben beschriebenen Bestandteile umfasst, in den Reaktor geleitet.

Die Erfindung wird durch die nachstehenden Beispiele näher erläutert.

### Beispiele

Die Beispiele wurden in einem Versuchsreaktor bestehend aus einem senkrecht stehenden Reaktionsrohr aus Edelstahl mit einem Innendurchmesser von 6 mm und einer Länge von 2300 mm durchgeführt. Das mit einem Mantel versehene Reaktionsrohr wurde mit heißem Öl der Temperatur T, das durch den Mantel strömte, beheizt. Mit sehr guter Nährung entspricht die Temperatur des Öls der Temperatur im Reaktionsrohr und somit der Reaktionstemperatur. Das Reaktionsrohr war von unten nach oben auf einer Höhe von ca. 200 mm mit inerten Steatitkugeln, darüber auf einer Höhe von ca. 1100 mm mit Katalysatorsplitt der Partikelgröße 0,5 - 1,0 mm und darüber auf einer Höhe von ca. 700 mm mit inerten Steatitkugeln gefüllt. Das Eingangsgas trat von oben in den Reaktor ein und am unteren Ende nach Passieren der Katalysatorschüttung wieder aus.

Das Eingangsgas bestand aus 7 Vol.-% O₂ und 35 Vol.-% C₂H₄ sowie 0,15 Vol.-% H₂O, 1,0 Vol.-% CO₂, Rest CH₄ (Methan). Die Versuche wurden bei p = 15 bar, einer Gasbelastung (GHSV) von 4750 h-1 sowie einer Raum-Zeit-Ausbeute an Ethylenoxid von 0,25 kgEO / LKat./h durchgeführt.

Die Reaktionstemperatur wurde gemäß der vorgegebenen Ethylenoxid-Raum-Zeit-Ausbeute geregelt. Zur Optimierung des Katalysators im Hinblick auf Selektivität und Umsatz wurden 3 Vol.-ppm Ethylchlorid als Moderator zum Feed zudosiert.

Das am Reaktorausgang austretende Gas wurde mittels Online-GC gaschromatographisch analysiert. Aus den Analyseergebnissen wurden der Umsatz (Katalysatoraktivität) und die Selektivität ermittelt. Bei vorgegebener Ethylenoxid-Raum-Zeit-Ausbeute war die hierzu notwendige Reaktionstemperatur ein Maß für die Katalysatoraktivität, wobei die Katalysatoraktivität dabei umgekehrt proportional zur Reaktionstemperatur ist.

Eine höhere Katalysatoraktivität wird dementsprechend durch eine niedrigere Reaktionstemperatur bei vorgegebener Ethylenoxid-Raum-Zeit-Ausbeute deutlich. Eine geringere Katalysatoraktivität wird umgekehrt durch eine höhere Reaktionstemperatur bei vorgegebener Ethylenoxid-Raum-Zeit-Ausbeute deutlich.

Der eingesetzte Silberkatalysator bestand aus ca. 15 Gew.-% Silber auf einem Trägermaterial aus alpha-Al₂O₃ mit geringen Anteilen SiO₂ sowie Spuren von Alkali- und Erdalkalimetalloxiden (spezifische Wasseraufnahme ca. 0,5 ml/g; spezifische BET Oberfläche von ca. 0,9 m²/g gemessen gemäß DIN-ISO 9277). Als selektivitätssteigemde Promotorelemente enthielt der Katalysator zusätzlich Lithium und Caesium sowie Wolfram und Schwefel.

Die Dosierung der Alkohole in flüssiger Form erfolgte mittels einer HPLC-Pumpe (Typ Bischoff mit Mikropumpenkopf 0 - 2 ml) unter Reaktionsdruck. Die Alkohole wurden dabei oberhalb des eigentlichen Reaktionsrohres in den Eingangsgasstrom dosiert und innerhalb einer ca. 1000 mm langen, auf ca. 175 °C beheizten Rohrwicklung aus Edelstahl auf 175 °C erwärmt. Die Alkohole wurden dabei in Abhängigkeit von Ihrer Siedetemperatur verdampft (im Falle von Methanol und Ethanol) oder in flüssiger Form (im Falle von 1-Butanol, 1-Propanol und 2-Propanol) vorgewärmt.

### Beispiel 1

### Einspeisung von Ethanol

Der Silberkatalysator wurde wie beschrieben in das Reaktionsrohr eingebaut. Zunächst wurde der Katalysator über eine Dauer von ca. 12 h drucklos bei 230 °C mit Stickstoff entsprechend einer GHSV von ca. 3000 h-1 gespült und so von Feuchtigkeit oder anderen anhaftenden Stoffen befreit. Das Anfahren des Katalysators erfolgte nach Erreichen des Reaktionsdruckes von 15 bar durch stufenweise Einstellung des oben beschriebenen Eingangsgasgemischs, wobei der Stickstoff stufenweise durch Methan als Inertgas ersetzt wurde. Nun wurde die Reaktionstemperatur so eingestellt, dass eine Raum-Zeit-Ausbeute an Ethylenoxid von 0,25 kg(EO) / I(Kat.) / h erzielt wurde.

Der Katalysator erreichte nach ca. 150 h Betriebszeit einen stationären Zustand, d. h. ein gleichbleibendes Aktivitäts- und Selektivitätsniveau. Nun erfolgte die Einspeisung von Ethanol, ohne dass dabei die übrigen Reaktionsbedingungen verändert wurden. Die Dosiergeschwindigkeit betrug 2,0 ml/h, entsprechend 0,3 Vol.-% Ethanol. Innerhalb von 60 min. wurde eine Ethanolmenge von 2 ml zudosiert. Unmittelbar nach der Zugabe kam es zu einem Rückgang der Katalysatorleistung. Nach einigen Stunden Betriebszeit wurde dann wieder ein stationäres Leistungsniveau des Katalysators bei vorgegebener Ethylenoxid-Raum-Zeit-Ausbeute erreicht. Die Ergebnisse sind in Tabelle 1 zusammengefasst.

**Tabelle 1: Katalysatorleistung vor, während und nach der Einspeisung von Ethanol**

| Bemerkung | Laufzeit (h) | Temperatur T (°C)* | Selektivität (%) |
|---|---|---|---|
| stationärer Zustand vor Einspeisung | 120 | 229 | 81,4 |
| unmittelbar nach Einspeisung | - | 229 | 78,9 |
| stationärer Zustand nach Einspeisung | 140 | 228 | 81,7 |

| | | | |
|---|---|---|---|
| * Die Temperatur ist umgekehrt proportional zur Katalysatoraktivität. | | | |

### Beispiel 2 (Vergleichsbeispiel)

### Einspeisung von Methanol

Das Beispiel 2 wurde analog zu Beispiel 1 durchgeführt, jedoch mit dem Unterschied, dass anstelle der Ethanol-Einspeisung die Einspeisung von Methanol erfolgte. Die Ergebnisse sind in Tabelle 2 zusammengefasst.

**Tabelle 2: Katalysatorleistung vor, während und nach der Einspeisung von Methanol**

| Bemerkung | Laufzeit (h) | Temperatur (°C)* | Selektivität (%) |
|---|---|---|---|
| stationärer Zustand vor Einspeisung | 120 | 248 | 81,8 |
| unmittelbar nach Einspeisung | - | 248 | 80,7 |
| stationärer Zustand nach Einspeisung | 150 | 248 | 82,0 |

| | | | |
|---|---|---|---|
| * Die Temperatur ist umgekehrt proportional zur Katalysatoraktivität. | | | |

### Beispiel 3 (Vergleichsbeispiel)

### Einspeisung von 1-Propanol

Das Beispiel 3 wurde analog zu Beispiel 1 durchgeführt, jedoch mit dem Unterschied, dass anstelle der Ethanol-Einspeisung die Einspeisung von 1-Propanol erfolgte. Die Ergebnisse sind in Tabelle 3 zusammengefasst.

**Tabelle 3: Katalysatorleistung vor, während und nach der Einspeisung von 1-Propanol**

| Bemerkung | Laufzeit (h) | Temperatur (°C)* | Selektivität (%) |
|---|---|---|---|
| stationärer Zustand vor Einspeisung | 160 | 228 | 81,5 |
| unmittelbar nach Einspeisung | - | 228 | 80,1 |
| stationärer Zustand nach Einspeisung | 200 | 228 | 81,3 |

| | | | |
|---|---|---|---|
| * Die Temperatur ist umgekehrt proportional zur Katalysatoraktivität | | | |

### Beispiel 4 (Vergleichsbeispiel)

### Einspeisung von 2-Propanol

Das Beispiel 4 wurde analog zu Beispiel 1 durchgeführt, jedoch mit dem Unterschied, dass anstelle der Ethanol-Einspeisung die Einspeisung von 2-Propanol erfolgte. Die Ergebnisse sind in Tabelle 4 zusammengefasst.

**Tabelle 4 Katalysatorleistung vor, während und nach der Einspeisung von 2-Propanol**

| Bemerkung | Laufzeit (h) | Temperatur (°C)* | Selektivität (%) |
|---|---|---|---|
| stationärer Zustand vor Einspeisung | 190 | 229 | 82,0 |
| unmittelbar nach Einspeisung | - | 229 | 80,2 |
| stationärer Zustand nach Einspeisung | 200 | 230 | 82,0 |

| | | | |
|---|---|---|---|
| * Die Temperatur ist umgekehrt proportional zur Katalysatoraktivität | | | |

### Beispiel 5 (Vergleichsbeispiel)

### Einspeisung von 1-Butanol

Das Beispiel 5 wurde analog zu Beispiel 1 durchgeführt, jedoch mit dem Unterschied, dass anstelle der Ethanol-Einspeisung die Einspeisung von 1-Butanol erfolgte. Die Ergebnisse sind in Tabelle 5 zusammengefasst.

**Tabelle 5: Katalysatorleistung vor, während und nach der Einspeisung von 1-Butanol**

| Bemerkung | Laufzeit (h) | Temperatur (°C)* | Selektivität (%) |
|---|---|---|---|
| stationärer Zustand vor Einspeisung | 170 | 226 | 81,6 |
| unmittelbar nach Einspeisung | - | 226 | 80,5 |
| stationärer Zustand nach Einspeisung | 190 | 226 | 81,9 |

| | | | |
|---|---|---|---|
| * Die Temperatur ist umgekehrt proportional zur Katalysatoraktivität | | | |

### Beispiel 6 (Vergleichsbeispiel)

### Keine Einspeisung eines Alkohols

Das Beispiel 6 wurde analog zu Beispiel 1 durchgeführt, jedoch mit dem Unterschied, dass keine Einspeisung eines Alkohols erfolgte. Die Ergebnisse sind in Tabelle 6 zusammengefasst.

**Tabelle 6: Katalysatorleistung zu bestimmten Laufzeiten ohne Einspeisung eines Alkohols**

| Bemerkung | Laufzeit (h) | Temperatur (°C)* | Selektivität (%) |
|---|---|---|---|
| stationärer Zustand | 125 | 228 | 81,4 |
| stationärer Zustand | 250 | 228 | 81,4 |

| | | | |
|---|---|---|---|
| * Die Temperatur ist umgekehrt proportional zur Katalysatoraktivität | | | |

Es ist zu erkennen, dass mit dem erfindungsgemäßen Verfahren die Aktivität, die ja umgekehrt proportional zur Reaktionstemperatur ist, und die Selektivität des Silberkatalysators beeinflusst werden. Es ist daher möglich, bei den erfindungsgemäß behandelten Katalysatoren bei gleicher Raum-Zeit-Ausbeute die Reaktionstemperatur nach der Behandlung zu senken. Dies ist auch insoweit von Vorteil, als mit niedriger Reaktionstemperatur die Bildung unerwünschter Nebenprodukte zurückgeht. Wie aus den Beispielen ersichtlich, wird im erfindungsgemäßen Beispiel 1 nach Behandlung eine Absenkung der Reaktionstemperatur (bei gleicher Raum-Zeit-Ausbeute) und eine Erhöhung der Selektivität erreicht. Bei keinem der Vergleichsversuche, bei denen das Verfahren mit von Ethanol verschiedenen Alkoholen durchgeführt wurde, konnte (bei gleicher Raum-Zeit-Ausbeute) eine solche Absenkung der Reaktionstemperatur erreicht werden.

## Patentansprüche

1. Kontinuierliches Verfahren zur Herstellung eines Alkylenoxids durch Direktoxidation eines Alkens mit Sauerstoff, umfassend
(i) kontinuierliches Inkontaktbringen eines Silber enthaltenden Katalysators mit einem Gemisch G1, enthaltend das Alken und Sauerstoff, für eine Laufzeit Δt(i);
(ii) zumindest einmaliges Inkontaktbringen des Katalysators gemäß (i) während des kontinuierlichen Inkontaktbringens gemäß (i) mit einem zusätzlichen Gemisch G2, enthaltend Ethanol, für eine Laufzeit Δt(ii),
wobei Δt(i) > Δt(ii) ist.

2. Verfahren gemäß Anspruch 1, wobei die Laufzeit Δt(i) mindestens 100 h beträgt.

3. Verfahren gemäß Anspruch 1 oder 2, wobei die Laufzeit Δt(ii) im Bereich von 0,1 bis 4 h liegt.

4. Verfahren gemäß einem der Ansprüche 1 bis 3, wobei das Inkontaktbringen gemäß (i) bei einer Temperatur im Bereich von 180 °C bis 300 °C, bevorzugt bei einer Temperatur im Bereich von 200 °C bis 270 °C, erfolgt.

5. Verfahren gemäß einem der Ansprüche 1 bis 4, wobei das Inkontaktbringen gemäß (i) bei einem Druck im Bereich von 14 bar bis 20 bar erfolgt.

6. Verfahren gemäß einem der Ansprüche 1 bis 5, wobei das Inkontaktbringen gemäß (i) mit einer GHSV (gas hourly space velocity) im Bereich von 3000 bis 6000 h⁻¹ erfolgt.

7. Verfahren gemäß einem der Ansprüche 1 bis 6, wobei das Gemisch G2 vor dem Inkontaktbringen mit dem Katalysator gemäß (ii) zum Gemisch G1 zudosiert wird.

8. Verfahren gemäß Anspruch 7, wobei das Gemisch G2 vor dem Zudosieren auf eine Temperatur im Bereich von 170 bis 270 °C gebracht wird.

9. Verfahren gemäß einem der Ansprüche 1 bis 8, wobei das Gemisch G2 in einer Menge im Bereich von 0,05 Vol.-% bis 1 Vol.-%, bevorzugt einer Menge im Bereich von 0,1 bis 0,5 Vol.-%, jeweils bezogen auf die Summe der Volumina G1 und G2, eingesetzt wird.

10. Verfahren gemäß einem der Ansprüche 1 bis 9, wobei das Alken Ethylen und das Alkylenoxid Ethylenoxid ist.

11. Verfahren gemäß einem der Ansprüche 1 bis 10, wobei das Gemisch G1 ein Inertgas enthält.

12. Verfahren gemäß Anspruch 11, wobei das Inertgas ausgewählt ist aus der Gruppe bestehend aus Stickstoff, Argon, Methan, Helium und Mischungen aus zwei oder mehr davon.

13. Verfahren gemäß einem der Ansprüche 1 bis 12, wobei das Gemisch G1 eine Halogenverbindung, bevorzugt eine organische Halogenverbindungen ausgewählt aus der Gruppe bestehend Ethylchlorid, 1,1-Dichloroethan, 1,2-Dichloroethan, Methylchlorid, Methylenchlorid, Vinylchlorid und Mischungen aus ein oder mehr dieser Verbindungen.

14. Verfahren gemäß einem der Ansprüche 1 bis 13, wobei das Gemisch G1 zusätzlich eine Stickstoffverbindung, bevorzugt Stickstoffmonoxid und/oder Stickstoffdioxid enthält.

15. Verfahren gemäß einem der Ansprüche 1 bis 14, wobei das Gemisch G2 zusätzlich Rheniumheptoxid enthält.

16. Verfahren gemäß einem der Ansprüche 1 bis 15, wobei der Silber enthaltende Katalysator ein inertes Trägermaterial, bevorzugt alpha-Aluminumoxid, umfasst.

17. Verfahren gemäß einem der Ansprüche 1 bis 16, wobei der Katalysator Silber in einer Menge von 10 bis 30 Gew.-%, bezogen auf das Gesamtgewicht des Katalysators, enthält.

18. Verfahren gemäß einem der Ansprüche 1 bis 17, wobei der Katalysator zusätzlich mindestens einen Promotor umfasst.

19. Verfahren gemäß Anspruch 18, wobei der mindestens eine Promotor ausgewählt ist aus der Gruppe bestehend aus Re, W, Mo, Rb, Li, K, Cs, Sr, Ba, Ca, P, B, In, Sn, Sb, TI, Pb, S, Bi und Mischungen aus zwei oder mehr davon.

## Claims

1. A continuous process for producing an alkylene oxide by direct oxidation of an alkene with oxygen, which comprises
(i)continuous contacting of a silver-comprising catalyst with a mixture G1 comprising the alkene and oxygen for a run time Δt(i);
(ii) contacting at least once the catalyst according to (i) during the continuous contacting according to (i) with an additional mixture G2 comprising ethanol for a run time Δt(ii),
wherein Δt(i) > Δt(ii).

2. The process according to claim 1, wherein the run time Δt(i) is at least 100 h.

3. The process according to claim 1 or 2, wherein the run time Δt(ii) is in the range from 0.1 to 4 h.

4. The process according to any of claims 1 to 3, wherein the contacting according to (i) proceeds at a temperature in the range from 180°C to 300°C, preferably at a temperature in the range from 200°C to 270°C.

5. The process according to any of claims 1 to 4, wherein the contacting according to (i) proceeds at a pressure in the range from 14 bar to 20 bar.

6. The process according to any of claims 1 to 5, wherein the contacting according to (i) proceeds with a gas hourly space velocity (GHSV) in the range from 3000 to 6000 h⁻¹.

7. The process according to any of claims 1 to 6, wherein the mixture G2, before the contacting with the catalyst according to (ii), is added to the mixture G1.

8. The process according to claim 7, wherein the mixture G2 is brought to a temperature in the range from 170 to 270°C before the addition.

9. The process according to any of claims 1 to 8, wherein the mixture G2 is used in an amount in the range from 0.05% by volume to 1% by volume, preferably an amount in the range from 0.1 to 0.5% by volume, in each case based on the sum of the volumes of G1 and G2.

10. The process according to any of claims 1 to 9, wherein the alkene is ethylene and the alkylene oxide is ethylene oxide.

11. The process according to any of claims 1 to 10, wherein the mixture G1 comprises an inert gas.

12. The process according to claim 11, wherein the inert gas is selected from the group consisting of nitrogen, argon, methane, helium and mixtures of two or more thereof.

13. The process according to any of claims 1 to 12, wherein the mixture G1 comprises a halogen compound, preferably an organic halogen compound selected from the group consisting of ethyl chloride, 1,1-dichloroethane, 1,2-dichloroethane, methyl chloride, methylene chloride, vinyl chloride and mixtures of one or more of these compounds.

14. The process according to any of claims 1 to 13, wherein the mixture G1 additionally comprises a nitrogen compound, preferably nitrogen monoxide and/or nitrogen dioxide.

15. The process according to any of claims 1 to 14, wherein the mixture G2 additionally comprises rhenium heptoxide.

16. The process according to any of claims 1 to 15, wherein the silver-comprising catalyst comprises an inert support material, preferably alpha-aluminum oxide.

17. The process according to any of claims 1 to 16, wherein the catalyst comprises silver in an amount of 10 to 30% by weight, based on the total weight of the catalyst.

18. The process according to any of claims 1 to 17, wherein the catalyst additionally comprises at least one promoter.

19. The process according to claim 18, wherein the at least one promoter is selected from the group consisting of Re, W, Mo, Rb, Li, K, Cs, Sr, Ba, Ca, P, B, In, Sn, Sb, Tl, Pb, S, Bi and mixtures of two or more thereof.

## Revendications

1. Procédé continu de fabrication d'un oxyde d'alkylène par oxydation directe d'un alcène avec de l'oxygène, comprenant :
(i) la mise en contact continue d'un catalyseur contenant de l'argent avec un mélange G1, contenant l'alcène et de l'oxygène, pendant une durée Δt(i) ;
(ii) la mise en contact au moins à une reprise du catalyseur selon (i) pendant la mise en contact continue selon (i) avec un mélange G2 supplémentaire contenant de l'éthanol, pendant une durée Δt(ii),
avec Δt(i) > Δt(ii).

2. Procédé selon la revendication 1, dans lequel la durée Δt(i) est d'au moins 100 h.

3. Procédé selon la revendication 1 ou 2, dans lequel la durée Δt(ii) se situe dans la plage allant de 0,1 à 4 h.

4. Procédé selon l'une quelconque des revendications 1 à 3, dans lequel la mise en contact selon (i) a lieu à une température dans la plage allant de 180 °C, à 300 °C, de préférence à une température dans la plage allant de 200 °C à 270 °C.

5. Procédé selon l'une quelconque des revendications 1 à 4, dans lequel la mise en contact selon (i) a lieu à une pression dans la plage allant de 14 bar à 20 bar.

6. Procédé selon l'une quelconque des revendications 1 à 5, dans lequel la mise en contact selon (i) a lieu avec une GHSV (gas hourly space velocity) dans la plage allant de 3 000 à 6 000 h⁻¹.

7. Procédé selon l'une quelconque des revendications 1 à 6, dans lequel le mélange G2 est ajouté au mélange G1 avant la mise en contact avec le catalyseur selon (ii).

8. Procédé selon la revendication 7, dans lequel le mélange G2 est porté à une température dans la plage allant de 170 à 270 °C avant l'ajout.

9. Procédé selon l'une quelconque des revendications 1 à 8, dans lequel le mélange G2 est utilisé en une quantité dans la plage allant de 0,05 % en volume à 1 % en volume, de préférence en une quantité dans la plage allant de 0,1 à 0,5 % en volume, à chaque fois par rapport à la somme des volumes de G1 et G2.

10. Procédé selon l'une quelconque des revendications 1 à 9, dans lequel l'alcène est l'éthylène et l'oxyde d'alkylène est l'oxyde d'éthylène.

11. Procédé selon l'une quelconque des revendications 1 à 10, dans lequel le mélange G1 contient un gaz inerte.

12. Procédé selon la revendication 11, dans lequel le gaz inerte est choisi dans le groupe constitué par l'azote, l'argon, le méthane, l'hélium et les mélanges de deux ou plus d'entre eux.

13. Procédé selon l'une quelconque des revendications 1 à 12, dans lequel le mélange G1 contient un composé halogéné, de préférence un composé halogéné organique choisi dans le groupe constitué par le chlorure d'éthyle, le 1,1-dichloroéthane, le 1,2-dichloroéthane, le chlorure de méthyle, le chlorure de méthylène, le chlorure de vinyle et les mélanges d'un ou de plusieurs de ces composés.

14. Procédé selon l'une quelconque des revendications 1 à 13, dans lequel le mélange G1 contient également un composé azoté, de préférence le monoxyde d'azote et/ou le dioxyde d'azote.

15. Procédé selon l'une quelconque des revendications 1 à 14, dans lequel le mélange G2 contient également de l'heptoxyde de rhénium.

16. Procédé selon l'une quelconque des revendications 1 à 15, dans lequel le catalyseur contenant de l'argent comprend un matériau support inerte, de préférence de l'oxyde d'aluminium alpha.

17. Procédé selon l'une quelconque des revendications 1 à 16, dans lequel le catalyseur contient de l'argent en une quantité de 10 à 30 % en poids, par rapport au poids total du catalyseur.

18. Procédé selon l'une quelconque des revendications 1 à 17, dans lequel le catalyseur comprend également au moins un promoteur.

19. Procédé selon la revendication 18, dans lequel le ou les promoteurs sont choisis dans le groupe constitué par Re, W, Mo, Rb, Li, K, Cs, Sr, Ba, Ca, P, B, In, Sn, Sb, Tl, Pb, S, Bi et les mélanges de deux ou plus d'entre eux.
